(19) Europäisches Patentamt
European Patent Office
Office européen des brevets

(11) **EP 3 280 460 B1**

(12) **EUROPÄISCHE PATENTSCHRIFT**

(45) Veröffentlichungstag und Bekanntmachung des Hinweises auf die Patenterteilung:
**29.11.2023 Patentblatt 2023/48**

(21) Anmeldenummer: **16712859.4**

(22) Anmeldetag: **29.03.2016**

(51) Internationale Patentklassifikation (IPC):
*A61L 15/22* (2006.01)    *A61L 15/60* (2006.01)
*C08F 265/04* (2006.01)    *C08F 265/06* (2006.01)
*C08F 6/18* (2006.01)    *C08J 11/02* (2006.01)

(52) Gemeinsame Patentklassifikation (CPC):
(C-Sets verfügbar)
**A61L 15/60; C08F 6/18; C08F 265/04; C08F 265/06; C08J 11/02**    (Forts.)

(86) Internationale Anmeldenummer:
**PCT/EP2016/056777**

(87) Internationale Veröffentlichungsnummer:
**WO 2016/162238 (13.10.2016 Gazette 2016/41)**

(54) **VERFAHREN ZUR HERSTELLUNG VON SUPERABSORBERPARTIKELN**

METHOD FOR PRODUCING SUPER ABSORBER PARTICLES

PROCÉDÉ DE PRODUCTION DE PARTICULES SUPERABSORBANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.04.2015 EP 15162597**

(43) Veröffentlichungstag der Anmeldung:
**14.02.2018 Patentblatt 2018/07**

(73) Patentinhaber: **BASF SE**
**67056 Ludwigshafen am Rhein (DE)**

(72) Erfinder:
• **MARK, Tina**
**67454 Hassloch (DE)**
• **DANIEL, Thomas**
**67165 Waldsee (DE)**
• **LUTZ, Erich**
**67122 Altrip (DE)**

(74) Vertreter: **BASF IP Association**
**BASF SE**
**G-FLP-C006**
**67056 Ludwigshafen (DE)**

(56) Entgegenhaltungen:
**EP-A1- 1 878 761    WO-A1-92/01008**
**WO-A1-2010/015591    WO-A1-2014/118024**

Bemerkungen:
Die Akte enthält technische Angaben, die nach dem Eingang der Anmeldung eingereicht wurden und die nicht in dieser Patentschrift enthalten sind.

(52) Gemeinsame Patentklassifikation (CPC): (Forts.)

C-Sets
**C08F 6/18, C08L 51/003;**
**C08F 265/04, C08F 265/02, C08F 220/06;**
**C08F 265/04, C08F 265/02, C08F 222/385;**
**C08F 265/06, C08F 265/02, C08F 220/06;**
**C08F 265/06, C08F 265/02, C08F 222/385**

**Beschreibung**

Die vorliegende Erfindung betrifft ein Verfahren zur Agglomeration von Superabsorberpartikeln, wobei Polymerpartikel mit einer Partikelgröße von 250 oder weniger $\mu$m in einem hydrophoben organischen Lösungsmittel dispergiert werden, die dispergierten Polymerpartikel mit einer wässrigen Monomerlösung gemischt werden, die mit der Monomerlösung aufgebrachte Menge an unneutralisiertem Monomer von 0,5 bis 80 Gew.-%, bezogen auf die dispergierten Polymerpartikel, beträgt und die Monomerlösung polymerisiert wird.

Die Herstellung von Superabsorberpartikeln wird in der Monographie "Modern Superabsorbent Polymer Technology", F.L. Buchholz und A.T. Graham, Wiley-VCH, 1998, Seiten 69 bis 117, beschrieben. Die Superabsorberpartikel werden üblicherweise durch Lösungspolymerisation oder Suspensionspolymerisation hergestellt.

Superabsorber werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität.

Zur Verbesserung der Anwendungseigenschaften, wie beispielsweise Permeabilität im gequollenen Gelbett in der Windel und Absorption unter Druck, werden Superabsorberpartikel im allgemeinen oberflächennachvernetzt. Dadurch steigt nur der Vernetzungsgrad der Partikeloberfläche, wodurch die Absorption unter Druck und die Zentrifugenretentionskapazität zumindest teilweise entkoppelt werden können.

EP 0 807 646 A1 beschreibt ein Verfahren zur Suspensionspolymerisation in Gegenwart von Polymerpartikeln.

WO 92/01008 A1 beschreibt ein Verfahren zur Rückführung abgetrennter, kleiner Polymerpartikel in die Polymerisation.

EP 1 878 761 A1 beschreibt eine Agglomeration in Gegenwart von Wasser.

WO 2005/092955 A1 beschreibt ein Verfahren zur Herstellung von Superabsorberpartikeln durch Lösungspolymerisation. Die Superabsorberpartikel können vor oder nach der Oberflächennachvernetzung unter Zusatz einer wässrigen Lösung agglomeriert werden, wobei der Wassergehalt der Superabsorberpartikel im Bereich von 1 bis 10 Gew.-% gehalten wird

WO 2006/014031 A1 beschreibt ein Verfahren zur Herstellung von Superabsorberpartikeln durch Suspensionspolymerisation. Die Superabsorberpartikel können vor oder nach der Oberflächennachvernetzung unter Zusatz einer wässrigen Lösung agglomeriert werden, wobei der Wassergehalt der Superabsorberpartikel im Bereich von 1 bis 10 Gew.-% gehalten wird.

WO 2010/015591 A1 beschreibt die Herstellung von Superabsorberpartikeln durch Sprühpolymerisation und WO 2014/118024 A1 beschreibt die Herstellung von Superabsorberpartikeln in einem Kneter.

Aufgabe der vorliegenden Erfindung war die Bereitstellung eines verbesserten Verfahrens zur Agglomeration von Superabsorberpartikeln, wobei die Superabsorberpartikel insbesondere eine hohe Absorptionskapazität und eine hohe Absorptionsgeschwindigkeit aufweisen sollen.

Eine weitere Aufgabe der vorliegenden Erfindung war die Bereitstellung eines verbesserten Verfahrens zur Agglomeration von Superabsorberpartikeln, wobei insbesondere aus anderen Verfahren abgetrennte zu kleine Polymerpartikel ("Fines") verwertet werden.

Gelöst wurde die Aufgabe durch ein Verfahren zur Agglomeration von Superabsorberpartikeln, wobei eine wässrige Monomerlösung 1, enthaltend

a) mindestens ein ethylenisch ungesättigtes, säuregruppentragendes Monomer, das zumindest teilweise neutralisiert sein kann,
b) optional einen oder mehrere Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

polymerisiert wird, die erhaltenen Polymerpartikel optional getrocknet und/oder zerkleinert werden, dadurch gekennzeichnet, dass Polymerpartikel mit einer Partikelgröße von 250 $\mu$m oder weniger abgetrennt werden, die abgetrennten Polymerpartikel in einem hydrophoben organischen Lösungsmittel dispergiert werden, die dispergierten Polymerpartikel mit einer wässrigen Monomerlösung 2, enthaltend

a) mindestens ein ethylenisch ungesättigtes, säuregruppentragendes Monomer, das zumindest teilweise neutralisiert sein kann,
b) optional einen oder mehrere Vernetzer,

c) mindestens einen Initiator,

d) optional ein oder mehrere mit den unter a) genannten Monomeren copolymerisierbare ethylenisch ungesättigte Monomere und

e) optional ein oder mehrere wasserlösliche Polymere,

gemischt werden, die Monomerlösung 1 und die Monomerlösung 2 gleich oder verschieden sein können, die mit der Monomerlösung 2 aufgebrachte Menge an unneutralisiertem Monomer a) von 0,5 bis 80 Gew.-%, bezogen auf die dispergierten Polymerpartikel, beträgt und die Monomerlösung 2 polymerisiert wird.

Es werden Polymerpartikel mit einer Partikelgröße von vorzugsweise 200 $\mu$m oder weniger, besonders bevorzugt 150 $\mu$m oder weniger, ganz besonders bevorzugt 120 $\mu$m oder weniger, abgetrennt. Die Partikelgröße können mittels eines Siebes mit der entsprechenden Maschenweite abgetrennt werden. Polymerpartikel, die ein Sieb mit einer Maschenweite von x $\mu$m passieren, weisen demnach eine Partikelgröße von x $\mu$m oder weniger auf.

Die Polymerpartikel können mittels eines Siebes mit einer Maschenweite von 250 $\mu$m oder weniger, vorzugsweise 200 $\mu$m oder weniger, besonders bevorzugt 150 $\mu$m oder weniger, ganz besonders bevorzugt 120 $\mu$m oder weniger, abgetrennt werden.

Die mit der Monomerlösung 2 aufgebrachte Menge an Monomer a) beträgt vorzugsweise von 1 bis 60 Gew.-%, besonders bevorzugt von 2 bis 40 Gew.-%, ganz besonders bevorzugt von 5 bis 30 Gew.-%, jeweils bezogen auf die Menge an dispergierten Polymerpartikeln.

Der vorliegenden Erfindung liegt die Erkenntnis zugrunde, dass die mit der Monomerlösung 2 aufgebrachte Menge an unneutralisiertem Monomer a) einen erheblichen Einfluss auf Zentrifugenretentionskapazität (CRC) und die Anquellgeschwindigkeit (FSR) der Agglomerate hat.

Im Folgenden wird die Herstellung der Superabsorberpartikel erläutert:

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) können teilweise neutralisiert sein. Die Neutralisation wird auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Methylenbisacrylamid, Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind Methylenbisacrylamid und die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Methylenbisacrylamid, Di- und/oder Triacrylate des 3- bis 10-fach ethoxylierten Glyzerins. Ganz besonders bevorzugt sind Methylenbisacrylamid, Di- oder Triacrylate des 1- bis 5-fach ethoxylierten und/oder propoxylierten Glyzerins. Am meisten bevorzugt sind Methylenbisacrylamid und die Triacrylate des 3- bis 5-fach ethoxylierten und/oder propoxylierten Glyzerins, insbesondere Methylenbisacrylamid und das Triacrylat des 3-fach ethoxylierten Glyzerins.

Die Menge an Vernetzer b) in der Monomerlösung 1 beträgt vorzugsweise 0,0001 bis 0,5 Gew.-%, besonders bevorzugt 0,001 bis 0,2 Gew.-%, ganz besonders bevorzugt 0,01 bis 0,05 Gew.-%, jeweils bezogen auf unneutralisiertes Monomer a). Mit steigendem Vernetzergehalt sinkt die Zentrifugenretentionskapazität (CRC) und die Absorption unter einem Druck von 21,0 $g/cm^2$ durchläuft ein Maximum.

Die Menge an Vernetzer b) in der Monomerlösung 2 beträgt vorzugsweise 0,01 bis 0,50 mmol, besonders bevorzugt 0,02 bis 0,25 mmol, ganz besonders bevorzugt 0,05 bis 0,15 mmol, jeweils bezogen auf 1 mol Monomer a). Mit fallendem Vernetzergehalt steigen Zentrifugenretentionskapazität (CRC) und Anquellgeschwindigkeit (FSR).

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren.

Geeignete Redox-Initiatoren sind Kalium- bzw. Natriumperoxodisulfat/Ascorbinsäure, Wasserstoffperoxid/Ascorbinsäure, Kalium- bzw. Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Kalium- bzw. Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoes-sigsäure und Natriumbisulfit eingesetzt. Derartige Gemische sind als Brüggolite® FF6 und Brüggolite® FF7 (Brüggemann Chemicals; Heilbronn; Deutschland) erhältlich.

Geeignete thermische Initiatoren sind insbesondere Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, 2,2'-Azobis(2-amidinopropan)dihydrochlorid, 4,4'-Azobis(4-cyanopentansäure), 4,4' und deren Natriumsalze, 2,2'-Azobis[2-methyl-N-(2-hydroxyethyl)propionamid] und 2,2'-Azobis(-imino-1-pyrrolidino-2-ethylpropans)dihydrochlorid.

Geeignete Photoinitiatoren sind beispielsweise 2-Hydroxy-2-methylpropiophenon und 1-[4-(2-Hydroxyethoxy)-phenyl]-2-hydroxy-2-methyl-1 -propan-1 -on.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Optional können der Monomerlösung oder ihren Ausgangsstoffen ein oder mehrere Chelatbildner zur Maskierung von Metallionen, wie beispielsweise Eisen, zwecks Stabilisierung zugesetzt werden. Geeignete Chelatbildner sind beispielsweise Alkalicitrate, Zitronensäure, Alkalitartrate, Pentanatriumtriphosphat, Ethylendiamintetraazetat, Nitrilotriessigsäure, sowie alle unter dem Namen Trilon® bekannten Chelatbildner, wie beispielsweise Trilon® C (Pentanatriumdiethylentriaminpentaazetat), Trilon® D (Trinatrium-(hydroxyethyl)-ethylen-diamintriazetat), sowie Trilon® M (Methylglycindiessigsäure).

Die Polymerisation der Monomerlösung 1 unterliegt keinen Beschränkungen. Es können alle bekannten Polymerisationsverfahren verwendet werden, beispielsweise die Lösungspolymerisation, die umgekehrte Suspensionspolymerisation und die Vertropfungspolymerisation.

Bei der Lösungspolymerisation wird die Monomerlösung 1 beispielsweise in einem Knetreaktor oder Bandreaktor polymerisiert. Im Knetreaktor wird das bei der Polymerisation der Monomerlösung 1 entstehende Polymergel durch beispielsweise gegenläufige Rührwellen kontinuierlich zerkleinert, wie in WO 2001/038402 A1 beschrieben. Die Polymerisation in einem Bandreaktor wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Anschließend wird das durch Lösungspolymerisation erhaltene Polymergel getrocknet, zerkleinert und klassiert. Dabei werden zu kleine Polymerpartikel ("Fines") abgetrennt. Dazu können Siebe mit einer Maschenweite von 250 µm oder weniger, vorzugsweise 200 µm oder weniger, besonders bevorzugt 150 µm oder weniger, ganz besonders bevorzugt 120 µm oder weniger, eingesetzt werden.

Bei der umgekehrten Suspensionspolymerisation wird die Monomerlösung 1 in einem hydrophoben Lösungsmittel suspendiert. Anschließend werden die erhaltenen Polymerpartikel azeotrop entwässert, durch Filtration vom Lösungsmittel getrennt und getrocknet.

Bei der umgekehrten Suspensionspolymerisation können ebenfalls zu kleine Polymerpartikel ("Fines") abgetrennt werden.

Bei der Vertropfungspolymerisation werden Tropfen der Monomerlösung 1 in einer umgebenden Gasphase polymerisiert. Hierbei können die Verfahrensschritte Polymerisation und Trocknung zusammengefasst werden, wie in WO 2008/040715 A2, WO 2008/052971 A1 und WO 2011/026876 A1 beschrieben. Zu kleine Polymerpartikel (Fines") werden mit dem Gasstrom mitgerissen und müssen daraus entfernt werden. Dazu können Filter oder Zyklone eingesetzt werden.

Die durch Polymerisation der Monomerlösung 1 erhaltenen und abgetrennten Polymerpartikel mit einer Partikelgröße von 250 µm oder weniger, vorzugsweise 200 µm oder weniger, besonders bevorzugt 150 µm oder weniger, ganz besonders bevorzugt 120 µm oder weniger, werden agglomeriert.

Zur Agglomeration werden die Polymerpartikel in einem hydrophoben Lösungsmittel dispergiert.

Als hydrophobe Lösungsmittel sind alle dem Fachmann zum Einsatz bei der Suspensionspolymerisation bekannten Lösungsmittel einsetzbar. Bevorzugt werden aliphatische Kohlenwasserstoffe, wie n-Hexan, n-Heptan, n-Oktan, n-Nonan, n-Dekan, Cyclohexan oder Mischungen daraus, verwendet. Hydrophobe Lösungsmittel weisen bei 23°C eine Löslichkeit in Wasser zu weniger als 5 g/100 g, vorzugsweise weniger als 1 g/100 g, besonders bevorzugt weniger als 0,5 g/100 g, auf.

Das hydrophobe Lösungsmittel siedet im Bereich von vorzugsweise 50 bis 150°C, besonders bevorzugt 60 bis 120°C, ganz besonders bevorzugt 70 bis 90°C.

Das Verhältnis zwischen hydrophoben Lösungsmittel und Polymerpartikel beträgt 0,2 bis 3,0 bevorzugt 0,3 bis 2,7 und ganz bevorzugt von 0,4 bis 2,4.

Zur Dispergierung der Polymerpartikel im hydrophoben Lösungsmittel bzw. zur Dispergierung der entstehenden Superabsorberpartikel können Dispergierhilfsmittel zugesetzt werden. Es kann sich dabei um anionische, kationische, nichtionische oder amphotere Tenside, oder natürliche, halbsynthetische oder synthetische Polymere handeln.

Anionische Tenside sind beispielsweise Natriumpolyoxyethylendodecylethersulfat und Natriumdodecylethersulfat. Ein kationisches Tensid ist beispielsweise Trimethylstearylammoniumchlorid. Ein amphoteres Tensid ist beispielsweise Carboxymethyldimethylcetylammonium. Nichtionische Tenside sind beispielsweise Saccharosefettsäureester, wie Saccharosemonostearat und Saccharosedilaurat, Sorbitanester, wie Sorbitanmonostearat, Trehalosefettsäureester, wie Trehalosestearinsäureester, Polyoxyalkylen-Verbindungen auf Basis von Sorbitanestern, wie Polyoxyethylensorbitanmonostearat.

Geeignete Polymere sind beispielsweise Zellulosederivate, wie Hydroxyethylzellulose, Methylhydroxyethylzellulose, Methylhydroxypropylcellulose, Methylzellulose und Carboxymethylzellulose, Polyvinylpyrrolidon, Copolymere des Vinylpyrrolidons, Gelatine, Gummiarabicum, Xanthan, Kasein, Polyglycerole, Polyglycerolfettsäureester, Polyethylenglykole, modifiziertes Polyethylenglykol, wie Polyethylenglykolstearat oder Polyethylenglykolstearyletherstearat Polyvinylalkohol, partiell hydrolysierte Polyvinylacetate und modifiziertes Polyethylen, wie ein mit Maleinsäure modifiziertes Polyethylen.

Es können aber auch anorganische Partikel als Dispergierhilfsmittel verwendet werden, sogenannte Pickering-Systeme. Ein solches Pickering-System kann dabei aus den festen Teilchen allein oder zusätzlich aus Hilfsstoffen bestehen, die die Dispergierbarkeit der Partikel in Wasser oder die Benetzbarkeit der Partikel durch das hydrophobe Lösungsmittel verbessern. Die Wirkweise und ihre Verwendung werden in WO 99/24525 A1 und EP 1 321 182 A1 beschrieben

Die anorganischen festen Partikel können Metallsalze sein, wie Salze, Oxide und Hydroxide von Kalzium, Magnesium, Eisen, Zink, Nickel, Titan, Aluminium, Silizium, Barium und Mangan. Zu nennen sind Magnesiumhydroxid, Magnesiumkarbonat, Magnesiumoxid, Kalziumoxalat, Kalziumkarbonat, Bariumkarbonat, Bariumsulfat, Titandioxid, Aluminiumoxid, Aluminiumhydroxid und Zinksulfid. Silikate, Bentonit, Hydroxyapatit und Hydrotalcite seien ebenfalls genannt. Besonders

bevorzugt sind auf SiO$_2$-basierende Kieselsäuren, Magnesiumpyrophosphat und Trikalziumphosphat.

Geeignete auf SiO$_2$-basierende Dispergierhilfsmittel sind hochdisperse Kieselsäuren. Sie können als feine, feste Teilchen in Wasser dispergiert werden. Es ist aber auch möglich, sogenannte kolloidale Dispersionen von Kieselsäure in Wasser zu verwenden. Solch kolloidale Dispersionen sind alkalische, wässrige Mischungen von Kieselsäure. Im alkalischen pH-Bereich sind die Partikel gequollen und in Wasser stabil. Bevorzugte kolloidale Dispersionen von Kieselsäure haben bei pH 9,3 eine spezifische Oberfläche im Bereich von 20 bis 90 m$^2$/g.

Weiterhin können beliebige Mischungen der Dispergierhilfsmittel eingesetzt werden.

Das Dispergierhilfsmittel wird üblicherweise im hydrophoben Lösungsmittel gelöst oder dispergiert. Das Dispergiermittel wird in Mengen zwischen 0.01 und 10 Gew.-%, bevorzugt zwischen 0,2 und 5 Gew.-%, besonders bevorzugt zwischen 0,5 und 2 Gew.-%, bezogen auf die Monomerlösung, eingesetzt.

Die Durchführung der Agglomeration ist dem Fachmann bekannt und unterliegt keinen Beschränkungen.

Zu den dispergierten Polymerpartikeln wird eine Monomerlösung 2 dosiert und erneut polymerisiert. Die Polymerpartikel agglomerieren erst bei der zweiten Polymerisation. Die Monomerlösung 1 und die Monomerlösung 2 können von ihrer Zusammensetzung identisch oder verschieden sein.

Mit jeder weiteren Monomerzugabe zu bereits gebildeten Agglomeraten können die Agglomerate weiter zu größeren Agglomeraten agglomeriert werden.

Zwischen den Monomerdosierungen können Kühlschritte liegen. Ein Teil des Dispergierhilfsmittels kann dabei ausfallen.

Vorteilhaft werden für die mehrstufige Agglomeration mehrere Rührreaktoren hintereinander geschaltet. Durch die Nachreaktion in weiteren Rührreaktoren kann der Monomerumsatz erhöht und die Rückvermischung vermindert werden.

Die Agglomeration wird vorzugsweise unter vermindertem Druck durchgeführt, beispielsweise bei einem Druck von 800 mbar. Über den Druck kann der Siedepunkt der Reaktionsmischung auf die gewünschte Reaktionstemperatur eingestellt werden.

In einer bevorzugten Ausführungsform der vorliegenden Erfindung werden die Superabsorberpartikel in der Polymerdispersion azeotrop entwässert, aus der Polymerdispersion filtriert und die filtrierten Superabsorberpartikel zur Entfernung des anhaftenden restlichen hydrophoben Lösungsmittels getrocknet.

Die erhaltenen Superabsorberpartikel können zur weiteren Verbesserung der Eigenschaften thermisch oberflächennachvernetzt werden. Die thermische Oberflächennachvernetzung kann in der Polymerdispersion oder mit den aus der Polymerdispersion abgetrennten und getrockneten Superabsorberpartikeln durchgeführt werden.

Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 Alkylenkarbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Weiterhin können auch Oberflächennachvernetzer eingesetzt werden, die zusätzliche polymerisierbare ethylenisch ungesättigte Gruppen enthalten, wie in DE 37 13 601 A1 beschrieben.

Weiterhin können beliebige Mischungen der geeigneten Oberflächennachvernetzer eingesetzt werden.

Bevorzugte Oberflächennachvernetzer sind Alkylenkarbonate, 2-Oxazolidinone, Bis- und Poly-2-oxazolidinone, 2-Oxotetrahydro-1,3-oxazine, N-Acyl-2-Oxazolidinone, zyklische Harnstoffe, bizyklische Amidoacetale, Oxetane, Bisoxetane und Morpholin-2,3-dione.

Besonders bevorzugte Oberflächennachvernetzer sind Ethylenkarbonat (1,3-Dioxolan-2-on), Trimethylenkarbonat (1,3-Dioxan-2-on), 3-Methyl-3-oxethanmethanol, 2-Hydroxyethyl-2-oxazolidinon, 2-Oxazolidinon und Methyl-2-oxazolidinon.

Ganz besonders bevorzugt ist Ethylenkarbonat.

Die Menge an Oberflächennachvernetzer beträgt vorzugsweise 0,1 bis 10 Gew.-%, besonders bevorzugt 0,5 bis 7,5 Gew.-%, ganz besonders bevorzugt 1 bis 5 Gew.-%, jeweils bezogen auf die Polymerpartikel.

Die Oberflächennachvernetzer werden typischerweise als wässrige Lösung eingesetzt. Die Menge des Lösungsmittels beträgt vorzugsweise 0,001 bis 8 Gew.-%, besonders bevorzugt 2 bis 7 Gew.-%, ganz besonders bevorzugt 3 bis 6 Gew.-%, und insbesondere 4 bis 5 Gew.-%, jeweils bezogen auf die Polymerpartikel. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasserund Propylenglykol/Wasser, wobei das Mischungsmassenverhältnis vorzugsweise von 10:90 bis 60:40 beträgt.

In einer bevorzugten Ausführungsform der vorliegenden Erfindung werden vor, während oder nach der thermischen Oberflächennachvernetzung zusätzlich zu den Oberflächennachvernetzern Kationen, insbesondere polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Karbonat, Hydrogenkarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch oberflächennachvernetzt.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Für die thermische Oberflächennachvernetzung kann es vorteilhaft sein, dieses im Unterdruck durchzuführen oder diesen unter Verwendung von Trocknungsgasen, wie beispielsweise getrocknete Luft und Stickstoff durchzuführen, um die möglichst vollständige Entfernung der Lösungsmittel zu gewährleisten.

Anschließend können die oberflächennachvernetzten Polymerpartikel klassiert werden, wobei zu kleine und/oder zu große Polymerpartikel abgetrennt und in das Verfahren rückgeführt werden.

Die Oberflächennachvernetzung kann auch in der Polymerdispersion durchgeführt werden. Dazu wird die Lösung des Oberflächennachvernetzers der Polymerdispersion zugesetzt. Dabei kann es vorteilhaft sein die thermische Oberflächennachvernetzung im Überdruck durchzuführen, beispielsweise bei Verwendung von hydrophoben organischen Lösungsmitteln mit einem Siedepunkt bei 1013 mbar unterhalb der gewünschten Temperatur für die thermische Oberflächennachvernetzung. Nach der thermischen Oberflächennachvernetzung in der Polymerdispersion werden die Superabsorberpartikel in der Polymerdispersion azeotrop entwässert, aus der Polymerdispersion abgetrennt, die abgetrennten Superabsorberpartikel zur Entfernung des anhaftenden restlichen hydrophoben Lösungsmittels getrocknet.

Bevorzugte Oberflächennachvernetzungstemperaturen liegen im Bereich 100 bis 220°C, vorzugsweise im Bereich von 105 bis 210°C, besonders bevorzugt im Bereich von 110 bis 205°C, ganz besonders bevorzugt im Bereich von 120 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 120 Minuten.

In einer weiteren bevorzugten Ausführungsform der vorliegenden Erfindung werden vor, während oder nach der thermischen Oberflächennachvernetzung zusätzlich Hydrophilisierungsmittel aufgebracht, beispielsweise Zuckeralkohole, wie Sorbitol, Mannitol und Xylitol, wasserlösliche Polymere oder Copolymere, wie Zellulose, Polyethylenglykole, Polyvinylalkohole, Polyvinylpyrrolidone und Polyacrylamide.

In einer bevorzugten Ausführungsform der vorliegenden Erfindung werden die Superabsorberpartikel nach der thermischen Oberflächennachvernetzung im Kontakttrockner gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Superabsorberpartikel auf 20 bis 150°C, vorzugsweise 30 bis 120°C, besonders bevorzugt 40 bis 100°C, ganz besonders bevorzugt 50 bis 80°C, abgekühlt.

Die im Kontakttrockner thermisch oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die Superabsorberpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20 und Plantacare 818 UP und Tensidmischungen.

Ein weiterer Gegenstand der vorliegenden Erfindung sind die gemäß dem erfindungsgemäßen Verfahren erhältlichen Superabsorberpartikel.

Die gemäß dem erfindungsgemäßen Verfahren erhältlichen Superabsorberpartikel weisen eine Zentrifugenretentionskapazität (CRC) von 35 bis 80 g/g, vorzugsweise von 36 bis 70 g/g, besonders bevorzugt von 37 bis 60 g/g, ganz besonders bevorzugt von 37,5 bis 55 g/g, und eine Anquellgeschwindigkeit (FSR) von 0,6 bis 1,6 g/g s, vorzugsweise von 0,7 bis 1,4 g/g s, besonders bevorzugt von 0,8 bis 1,2 g/g s, ganz besonders bevorzugt von 0,85 bis 1,1 g/g s, auf.

Die gemäß dem erfindungsgemäßen Verfahren erhältlichen Superabsorberpartikel weisen vorzugsweise eine Zentrifugenretentionskapazität (CRC) von 36 bis 70 g/g und eine Anquellgeschwindigkeit (FSR) von 0,7 bis 1,4 g/g s auf.

Die gemäß dem erfindungsgemäßen Verfahren erhältlichen Superabsorberpartikel weisen besonders bevorzugt eine Zentrifugenretentionskapazität (CRC) von 37 bis 60 g/g und eine Anquellgeschwindigkeit (FSR) von 0,8 bis 1,2 g/g s auf.

Die gemäß dem erfindungsgemäßen Verfahren erhältlichen Superabsorberpartikel weisen ganz besonders bevorzugt eine Zentrifugenretentionskapazität (CRC) von 37,5 bis 55 g/g und eine Anquellgeschwindigkeit (FSR) von 0,85 bis 1,1 g/g s auf.

Ein weiterer Gegenstand der vorliegenden Erfindung sind Hygieneartikel, umfassend

(A) eine obere flüssigkeitsdurchlässige Schicht,
(B) eine untere flüssigkeitsundurchlässige Schicht,
(C) eine flüssigkeitsabsorbierende Speicherschicht zwischen der Schicht (A) und der Schicht (B), enthaltend von 0 bis 30 Gew.-% eines Fasermaterial und von 70 bis 100 Gew.-% gemäß dem erfindungsgemäßen Verfahren erhältliche Superabsorberpartikel,
(D) optional eine Aufnahme- und Verteilschicht zwischen der Schicht (A) und der Schicht (C), enthaltend von 80 bis 100 Gew.-% eines Fasermaterial und von 0 bis 20 Gew.-% gemäß dem erfindungsgemäßen Verfahren erhältliche Superabsorberpartikel,
(E) optional eine Gewebeschicht direkt über und/oder unter der Schicht (C) und
(F) weitere optionale Komponenten.

Der Anteil an gemäß dem erfindungsgemäßen Verfahren erhältlichen Superabsorberpartikel in der flüssigkeitsabsorbierenden Speicherschicht (C) beträgt vorzugsweise mindestens 75 Gew.-%, besonders bevorzugt mindestens 80 Gew.-%, ganz besonders bevorzugt mindestens 90 Gew.-%.

Die mittlere Sphärizität der gemäß dem erfindungsgemäßen Verfahren erhältlichen Superabsorberpartikel in der flüssigkeitsabsorbierenden Speicherschicht (C) beträgt vorzugsweise weniger als 0,84, besonders bevorzugt weniger als 0,82, ganz besonders bevorzugt weniger als 0,80.

Superabsorberpartikel mit relativ niedriger Sphärizität werden erhalten, wenn die Polymerpartikel agglomeriert werden. In den erfindungsgemäßen Hygieneartikeln werden agglomerierte Superabsorberpartikel eingesetzt.

Die Superabsorberpartikel werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

Feuchtegehalt

Der Feuchtegehalt der Superabsorberpartikel wird gemäß der von der EDANA empfohlenen Testmethode Nr. WSP 230.3 (11) "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.3 (11) "Fluid Retention Capacity in Saline, After Centrifugation" bestimmt.

Anquellgeschwindigkeit (Free Swell Rate)

Zur Bestimmung der Anquellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

$$FSR \ [g/g \ s] = W2/(W1xt)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Partikelgrößenverteilung

Die mittlere Partikelgröße und die Breite der Partikelgrößenverteilung ($\sigma_\zeta$) wurden analog zu EP 0 349 240 B1 ermittelt, wobei Siebe mit Maschenweiten von $100\mu m$, $200\mu m$, $300\mu m$, $400\mu m$, $450\mu m$, $500\mu m$, $600\mu m$, $710\mu m$, $800\mu m$, $900\mu m$, $1000\mu m$, $1180\mu m$, $1400\mu m$, $1600\mu m$, $1700\mu m$, $2000\mu m$ und $4000 \ \mu m$ eingesetzt werden.

Die Werte für die Partikelgrößenverteilung $\sigma_\zeta$ sind umso niedriger, je enger die Partikelgrößenverteilung ist.

mittlere Sphärizität (mSPHT)

Die mittlere Sphärizität (mSPHT) wird mit dem PartAn® 3001 L Partikel Analysator (Microtrac Europe GmbH; DE) bestimmt.

Die zu analysierende Probe wird in einen Trichter eingefüllt. Das rechnergesteuerte Messsystem startet die Dosiervorrichtung und sorgt für einen kontinuierlichen, konzentrationsgeregelten Partikelstrom. Die Partikel fallen vereinzelt durch den Messschacht und erzeugen kontrastreiche Schattenbilder zwischen Lichtquelle und hochauflösender Kamera. Die Lichtquelle wird von der Kamera angesteuert und erzeugt aufgrund sehr kurzer Belichtungszeiten fehlerfreie Bildinformationen für die Mehrfach-Auswertung jedes einzelnen Partikels in Echtzeit.

In einem 3D-Verfahren wird jedes Partikel mehrfach analysiert und das Verfahren liefert so die absoluten Ergebnisse zur Länge, Breite, Dicke, Fläche und Umfang. Über die Anzahl der vom Partikel abgedeckten Pixel wird die Größe und Form berechnet. Daraus resultiert auch die präzisere Bestimmung der mittleren Sphärizität (mSPHT).

Beispiel 1

In ein 2 L-Planschliffgefäß, ausgestattet mit Impellerrüher und Rückflusskühler, wurden 500,00 g Heptan, 0,92 g Saccharosestearat (Ryoto® Sugar Ester S-370, Mitsubishi Chemical Europe GmbH, Düsseldorf, Deutschland) und 60,00 g Superabsorberpartikel vorgelegt und unter Rühren und Stickstoffeinleitung auf 70°C erhitzt bis das Saccharosestearat vollständig gelöst war. Die Superabsorberpartikel wurden gemäß Example 1 von WO 2014/079694 A1 hergestellt und aus dem Strom (8) in Figur 1 abgeschieden.

Eine Monomerlösung 2, hergestellt aus 50,00 g (0,694 mol) Acrylsäure, 41,63 g (0,520 mol), 50gew.-%iger wässriger Natriumhydroxidlösung, 46,71 g Wasser, 0,0125 g (0,081 mmol) N,N'-Methylenbisacrylamid (MBA) und 0,167 g (0,618 mmol) Kaliumperoxodisulfat, wurde in ein Zulaufgefäß gefüllt und mit Luft gespült. Die Monomerlösung 2 wurde innerhalb von 30 Minuten bei einer Rührerdrehzahl von 400 U/min zugetropft. Die Monomerlösung 2 wurde unmittelbar vor dem Zutropfen mit Stickstoff inertisiert.

Nach Zulaufende wurde weitere 60 Minuten bei 70°C gerührt. Anschließend wurde der Rückflusskühler gegen einen Wasserauskreiser ausgetauscht und Wasser ausgekreist.

Die vorliegende Suspension wurde auf 60°C abgekühlt und die erhaltenen Polymerpartikel wurden über einen Büchnertrichter mit Papierfilter abgesaugt. Die weitere Trocknung erfolgte bei 45°C im Umlufttrockenschrank und ggf. im Vakuumtrockenschrank bei 800 mbar bis zu einem Restfeuchtegehalt von kleiner 15 Gew.-%.

Die Eigenschaften der erhaltenen Polymerpartikel sind in den Tabellen 1 und 2 zusammengefasst.

Beispiel 2

Es wurde verfahren wie unter Beispiel 1, wobei eine Monomerlösung 2, hergestellt aus 12,50 g (0,173 mol) Acrylsäure, 10,41 g (0,130 mol), 50gew.-%iger wässriger Natriumhydroxidlösung, 18,62 g Wasser, 0,0031 g (0,020 mmol) N,N'-Methylenbisacrylamid (MBA) und 0,019 g (0,069 mmol) Kaliumperoxodisulfat, verwendet wurde.

Die Eigenschaften der erhaltenen Polymerpartikel sind in den Tabellen 1 und 2 zusammengefasst.

Beispiel 3

Es wurde verfahren wie unter Beispiel 1, wobei eine Monomerlösung 2, hergestellt aus 12,50 g (0,173 mol) Acrylsäure, 10,41 g (0,130 mol), 50gew.-%iger wässriger Natriumhydroxidlösung, 18,62 g Wasser, 0,0063 g (0,041 mmol) N,N'-Methylenbisacrylamid (MBA) und 0,019 g (0,069 mmol) Kaliumperoxodisulfat, verwendet wurde.

Die Eigenschaften der erhaltenen Polymerpartikel sind in den Tabellen 1 und 2 zusammengefasst.

Beispiel 4

Es wurde verfahren wie unter Beispiel 1, wobei eine Monomerlösung 2, hergestellt aus 12,50 g (0,173 mol) Acrylsäure, 10,41 g (0,130 mol), 50gew.-%iger wässriger Natriumhydroxidlösung, 18,62 g Wasser, 0,0125 g (0,081 mmol) N,N'-Methylenbisacrylamid (MBA) und 0,019 g (0,069 mmol) Kaliumperoxodisulfat, verwendet wurde.

Die Eigenschaften der erhaltenen Polymerpartikel sind in den Tabellen 1 und 2 zusammengefasst.

Tab. 1: Eigenschaften der agglomerierten Superabsorber

| Beispiel | AS Gew.-% bop | MBA mmol boa | CRC g/g | FSR g/gs | Feuchte Gew.-% |
|---|---|---|---|---|---|
| 1*) | 83 | 0,46 | 24,7 | 0,76 | 4,1 |
| 2 | 21 | 0,12 | 38,0 | 0,81 | 5,1 |
| 3 | 21 | 0,24 | 36,4 | 0,76 | 4,8 |
| 4 | 21 | 0,46 | 33,8 | 0,76 | 4,7 |
| AS Acrylsäure<br>bop bezogen auf Superabsorberpartikel (based on polymer)<br>MBA N,N'-Methylenbisacrylamid<br>boa bezogen auf unneutralisierte Acrylsäure (based on acrylic acid)<br>*) Vergleichsbeispiel | | | | | |

Tab. 2: Siebanalyse (Gew.-%)

| Bsp. | <100 µm | 100-200 µm | 200-300 µm | 300-400 µm | 400-500 µm | 500-600 µm | 600-700 µm | 700-800 µm | 800-900 µm | 900-1000 µm | >1000 µm |
|---|---|---|---|---|---|---|---|---|---|---|---|
| 1*) | 1 | 2 | 5 | 8 | 9 | 6 | 10 | 7 | 7 | 5 | 40 |
| 2 | 2 | 13 | 27 | 24 | 12 | 4 | 4 | 2 | 2 | 2 | 8 |
| 3 | 1 | 8 | 20 | 22 | 14 | 6 | 7 | 3 | 3 | 2 | 12 |
| 4 | 2 | 8 | 20 | 24 | 16 | 7 | 8 | 3 | 2 | 2 | 8 |

*) Vergleichsbeispiel

**Patentansprüche**

1. Verfahren zur Agglomeration von Superabsorberpartikeln, wobei eine wässrige Monomerlösung 1, enthaltend

    a) mindestens ein ethylenisch ungesättigtes, säuregruppentragendes Monomer, das zumindest teilweise neutralisiert sein kann,
    b) optional einen oder mehrere Vernetzer,
    c) mindestens einen Initiator,
    d) optional ein oder mehrere mit den unter a) genannten Monomeren copolymerisierbare ethylenisch ungesättigte Monomere und
    e) optional ein oder mehrere wasserlösliche Polymere,

    polymerisiert wird, die erhaltenen Polymerpartikel optional getrocknet und/oder zerkleinert werden, **dadurch gekennzeichnet, dass** Polymerpartikel mit einer Partikelgröße von 250 $\mu$m oder weniger abgetrennt werden, die abgetrennten Polymerpartikel in einem hydrophoben organischen Lösungsmittel dispergiert werden, das hydrophobe organische Lösungsmittel bei 23°C eine Löslichkeit in Wasser von weniger als 5 g/100 g aufweist, die dispergierten Polymerpartikel mit einer wässrigen Monomerlösung 2, enthaltend

    a) mindestens ein ethylenisch ungesättigtes, säuregruppentragendes Monomer, das zumindest teilweise neutralisiert sein kann,
    b) optional einen oder mehrere Vernetzer,
    c) mindestens einen Initiator,
    d) optional ein oder mehrere mit den unter a) genannten Monomeren copolymerisierbare ethylenisch ungesättigte Monomere und
    e) optional ein oder mehrere wasserlösliche Polymere,

    gemischt werden, die Monomerlösung 1 und die Monomerlösung 2 gleich oder verschieden sein können, die mit der Monomerlösung 2 aufgebrachte Menge an unneutralisiertem Monomer a) von 0,5 bis 80 Gew.-%, bezogen auf die dispergierten Polymerpartikel, beträgt und die Monomerlösung 2 polymerisiert wird.

2. Verfahren gemäß Anspruch 1, **dadurch gekennzeichnet, dass** Polymerpartikel mit einer Partikelgröße von 150 $\mu$m oder weniger abgetrennt werden.

3. Verfahren gemäß Anspruch 1 oder 2, **dadurch gekennzeichnet, dass** die mit der Monomerlösung 2 aufgebrachte Menge an unneutralisiertem Monomer a) von 5 bis 30 Gew.-%, bezogen auf die dispergierten Polymerpartikel, beträgt.

4. Verfahren gemäß einem der Ansprüche 1 bis 3, **dadurch gekennzeichnet, dass** die Monomerlösung 2 von 0,01 bis 0,50 mmol Vernetzer b), bezogen auf unneutralisiertes Monomer a), enthält.

5. Verfahren gemäß einem der Ansprüche 1 bis 3, **dadurch gekennzeichnet, dass** die Monomerlösung 2 von 0,05 bis 0,15 mmol Vernetzer b), bezogen auf unneutralisiertes Monomer a), enthält.

6. Verfahren gemäß einem der Ansprüche 1 bis 5, **dadurch gekennzeichnet, dass** die Monomerlösung 1 durch Vertropfungspolymerisation polymerisiert wird.

7. Verfahren gemäß Anspruch 6, **dadurch gekennzeichnet, dass** die Polymerpartikel mittels eines Filters oder Zyklons aus dem Abgas der Vertropfungspolymerisation abgetrennt werden.

8. Verfahren gemäß einem der Ansprüche 1 bis 5, **dadurch gekennzeichnet, dass** die Monomerlösung 1 durch Suspensionspolymerisation polymerisiert wird und die erhaltenen Polymerpartikel getrocknet werden.

9. Verfahren gemäß einem der Ansprüche 1 bis 5, **dadurch gekennzeichnet, dass** die Monomerlösung 1 durch Lösungspolymerisation polymerisiert wird und die erhaltenen Polymerpartikel getrocknet und zerkleinert werden.

10. Verfahren gemäß Anspruch 8 oder 9, **dadurch gekennzeichnet, dass** die Polymerpartikel mittels eines Siebes mit einer Maschenweite von 250 $\mu$m oder weniger abgetrennt werden.

11. Verfahren gemäß Anspruch 8 oder 9, **dadurch gekennzeichnet, dass** die Polymerpartikel mittels eines Siebes mit einer Maschenweite von 150 μm oder weniger abgetrennt werden.

12. Agglomerierte Superabsorberpartikel, erhältlich nach einem Verfahren der Ansprüche 1 bis 11, wobei die Superabsorberpartikel eine Zentrifugenretentionskapazität von 35 bis 80 g/g und eine Anquellgeschwindigkeit von 0,6 bis 1,6 g/g s aufweisen.

13. Agglomerierte Superabsorberpartikel gemäß Anspruch 12, wobei die Superabsorberpartikel eine Zentrifugenretentionskapazität von 36 bis 70 g/g und eine Anquellgeschwindigkeit von 0,7 bis 1,4 g/g s aufweisen.

14. Agglomerierte Superabsorberpartikel gemäß Anspruch 12 oder 13, wobei die Superabsorberpartikel eine Zentrifugenretentionskapazität von 37 bis 60 g/g und eine Anquellgeschwindigkeit von 0,8 bis 1,2 g/g s aufweisen.

15. Hygieneartikel, umfassend

(A) eine obere flüssigkeitsdurchlässige Schicht,
(B) eine untere flüssigkeitsundurchlässige Schicht,
(C) eine flüssigkeitsabsorbierende Speicherschicht zwischen der Schicht (A) und der Schicht (B), enthaltend von 0 bis 30 Gew.-% eines Fasermaterial und von 70 bis 100 Gew.-% agglomerierte Superabsorberpartikel gemäß einem der Ansprüche 12 bis 14,
(D) optional eine Aufnahme- und Verteilschicht zwischen der Schicht (A) und der Schicht (C), enthaltend von 80 bis 100 Gew.-% eines Fasermaterial und von 0 bis 20 Gew.-% agglomerierte Superabsorberpartikel I gemäß einem der Ansprüche 12 bis 14,
(E) optional eine Gewebeschicht direkt über und/oder unter der Schicht (C) und
(F) weitere optionale Komponenten.

## Claims

1. A process for agglomerating superabsorbent particles by polymerizing an aqueous monomer solution 1 comprising

a) at least one ethylenically unsaturated monomer which bears acid groups and may have been at least partly neutralized,
b) optionally one or more crosslinkers,
c) at least one initiator,
d) optionally one or more ethylenically unsaturated monomers copolymerizable with the monomers mentioned under a) and
e) optionally one or more water-soluble polymers,

and optionally drying and/or comminuting the resulting polymer particles, wherein polymer particles having a particle size of 250 μm or less are removed, the polymer particles removed are dispersed in a hydrophobic organic solvent, the hydrophobic organic solvent at 23°C has a solubility in water of less than 5 g/100 g, the dispersed polymer particles are mixed with an aqueous monomer solution 2 comprising

a) at least one ethylenically unsaturated monomer which bears acid groups and may have been at least partly neutralized,
b) optionally one or more crosslinkers,
c) at least one initiator,
d) optionally one or more ethylenically unsaturated monomers copolymerizable with the monomers mentioned under a) and
e) optionally one or more water-soluble polymers,

monomer solution 1 and monomer solution 2 may be the same or different, the amount of unneutralized monomer a) applied with monomer solution 2 is from 0.5% to 80% by weight, based on the dispersed polymer particles, and monomer solution 2 is polymerized.

2. The process according to claim 1, wherein polymer particles having a particle size of 150 μm or less are removed.

3. The process according to claim 1 or 2, wherein the amount of unneutralized monomer a) applied with monomer solution 2 is from 5% to 30% by weight, based on the dispersed polymer particles.

4. The process according to any of claims 1 to 3, wherein monomer solution 2 comprises from 0.01 to 0.50 mmol of crosslinker b), based on unneutralized monomer a).

5. The process according to any of claims 1 to 3, wherein monomer solution 2 comprises from 0.05 to 0.15 mmol of crosslinker b), based on unneutralized monomer a).

6. The process according to any of claims 1 to 5, wherein monomer solution 1 is polymerized by dropletizing polymerization.

7. The process according to claim 6, wherein the polymer particles are removed from the offgas of the dropletizing polymerization by means of a filter or cyclone.

8. The process according to any of claims 1 to 5, wherein monomer solution 1 is polymerized by suspension polymerization and the resulting polymer particles are dried.

9. The process according to any of claims 1 to 5, wherein monomer solution 1 is polymerized by solution polymerization and the resulting polymer particles are dried and comminuted.

10. The process according to claim 8 or 9, wherein the polymer particles are removed by means of a sieve having a mesh size of 250 $\mu$m or less.

11. The process according to claim 8 or 9, wherein the polymer particles are removed by means of a sieve having a mesh size of 150 $\mu$m or less.

12. Agglomerated superabsorbent particles obtainable by a process of claims 1 to 11, having a centrifuge retention capacity of 35 to 80 g/g and a free swell rate of 0.6 to 1.6 g/g s.

13. Agglomerated superabsorbent particles according to claim 12, having a centrifuge retention capacity of 36 to 70 g/g and a free swell rate of 0.7 to 1.4 g/g s.

14. Agglomerated superabsorbent particles according to claim 12 or 13, having a centrifuge retention capacity of 37 to 60 g/g and a free swell rate of 0.8 to 1.2 g/g s.

15. A hygiene article comprising

(A) an upper liquid-permeable layer,
(B) a lower liquid-impermeable layer,
(C) a liquid-absorbing storage layer between layer (A) and layer (B), comprising from 0% to 30% by weight of a fibrous material and from 70% to 100% by weight of agglomerated superabsorbent particles according to any of claims 12 to 14,
(D) optionally an acquisition and distribution layer between layer (A) and layer (C), comprising from 80% to 100% by weight of a fibrous material and from 0 to 20% by weight of agglomerated superabsorbent particles according to any of claims 12 to 14,
(E) optionally a fabric layer directly above and/or beneath layer (C) and
(F) further optional components.

**Revendications**

1. Procédé d'agglomération de particules superabsorbantes, dans lequel on polymérise une solution aqueuse de polymères 1, contenant

a) au moins un monomère portant un groupe acide, à insaturation éthylénique, qui peut au moins partiellement être neutralisé,
b) éventuellement un ou plusieurs réticulants,

c) au moins un amorceur,

d) éventuellement un ou plusieurs monomères à insaturation éthylénique, copolymérisables avec les monomères mentionnés en a) et

e) éventuellement un ou plusieurs polymères solubles dans l'eau,

on sèche et/ou on broie éventuellement les particules polymères obtenues, **caractérisé en ce que** les particules polymères sont séparées avec une granulométrie de 250 μm ou moins, les particules polymères séparées sont dispersées dans un solvant organique hydrophobe, le solvant organique hydrophobe présente à 23 °C une solubilité dans l'eau inférieure à 5 g/100 g, les particules polymères dispersées sont mélangées à une solution aqueuse de monomères 2 contenant

a) au moins un monomère portant un groupe acide, à insaturation éthylénique, qui peut au moins partiellement être neutralisé,

b) éventuellement un ou plusieurs réticulants,

c) au moins un amorceur,

d) éventuellement un ou plusieurs monomères à insaturation éthylénique, copolymérisables avec les monomères mentionnés en a) et

e) éventuellement un ou plusieurs polymères solubles dans l'eau,

la solution de monomères 1 et la solution de monomères 2 peuvent être identiques ou différentes, la quantité du monomère non neutralisé a) appliquée avec la solution de monomères 2 est de 0,5 à 80 % en poids par rapport aux particules polymères dispersées, et la solution de monomères 2 est polymérisée.

2. Procédé selon la revendication 1, **caractérisé en ce que** les particules polymères sont séparées avec une granulométrie de 150 μm ou moins.

3. Procédé selon la revendication 1 ou 2, **caractérisé en ce que** la quantité du monomère non neutralisé a) appliquée avec la solution de monomères 2 est de 5 à 30 % en poids par rapport aux particules polymères dispersées.

4. Procédé selon l'une des revendications 1 à 3, **caractérisé en ce que** la solution de monomères 2 contient 0,01 à 0,50 mmol du réticulant b), par rapport au monomère a) non neutralisé.

5. Procédé selon l'une des revendications 1 à 3, **caractérisé en ce que** la solution de monomères 2 contient 0,05 à 0,15 mmol du réticulant b), par rapport au monomère a) non neutralisé.

6. Procédé selon l'une des revendications 1 à 5, **caractérisé en ce que** la solution de monomères 1 est polymérisée par polymérisation par formation de gouttes.

7. Procédé selon la revendication 6, **caractérisé en ce que** les particules polymères sont séparées à l'aide d'un filtre ou d'un cyclone à partir de l'effluent gazeux de la polymérisation par formation de gouttes.

8. Procédé selon l'une des revendications 1 à 5, **caractérisé en ce que** la solution de monomères 1 est polymérisée par polymérisation en suspension, et les particules polymères obtenues sont séchées.

9. Procédé selon l'une des revendications 1 à 5, **caractérisé en ce que** la solution de monomères 1 est polymérisée par polymérisation en solution et les particules polymères obtenues sont séchées et broyées.

10. Procédé selon la revendication 8 ou 9, **caractérisé en ce que** les particules polymères sont séparées à l'aide d'un tamis ayant une ouverture de maille de 250 μm ou moins.

11. Procédé selon la revendication 8 ou 9, **caractérisé en ce que** les particules polymères sont séparées à l'aide d'un tamis ayant une ouverture de maille de 150 μm ou moins.

12. Particules superabsorbantes agglomérées pouvant être obtenues par un procédé selon les revendications 1 à 11, les particules superabsorbantes présentant une capacité de rétention centrifuge de 35 à 80 g/g et une vitesse de gonflement de 0,6 à 1,6 g/g·s.

13. Particules superabsorbantes agglomérées selon la revendication 12, les particules superabsorbantes présentant une capacité de rétention centrifuge de 36 à 70 g/g et une vitesse de gonflement de 0,7 à 1,4 g/g·s.

14. Particules superabsorbantes agglomérées selon la revendication 12 ou 13, les particules superabsorbantes présentant une capacité de rétention centrifuge de 37 à 60 g/g et une vitesse de gonflement de 0,8 à 1,2 g/g·s.

15. Article d'hygiène, comprenant

(A) une couche supérieure perméable aux liquides,
(B) une couche inférieure imperméable aux liquides,
(C) une couche de stockage absorbant les liquides entre la couche (A) et la couche (B), contenant de 0 à 30 % en poids d'un matériau fibreux et 70 à 100 % en poids de particules superabsorbantes agglomérées selon l'une des revendications 12 à 14,
(D) éventuellement, une couche de réception et de répartition entre la couche (A) et la couche (C), contenant de 80 à 100 % en poids d'un matériau fibreux et de 0 à 20 % en poids de particules superabsorbantes agglomérées selon l'une des revendications 12 à 14,
(E) éventuellement une couche de tissu directement sur et/ou sous la couche (C) et
(F) d'autres composants éventuels.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0807646 A1
- WO 9201008 A1
- EP 1878761 A1
- WO 2005092955 A1
- WO 2006014031 A1
- WO 2010015591 A1
- WO 2014118024 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1

- US 6241928 B
- WO 2008040715 A2
- WO 2008052971 A1
- WO 2011026876 A1
- WO 9924525 A1
- EP 1321182 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0349240 B1
- WO 2014079694 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 69-117